# EUROPÄISCHE PATENTSCHRIFT

(11) **EP 0 679 370 B1**
(45) Veröffentlichungstag und Bekanntmachung des Hinweises auf die Patenterteilung: **08.03.2000**
(21) Anmeldenummer: 95106496.3
(22) Anmeldetag: 28.04.1995
(51) Int. Cl.: A61B 17/70

(54) **Knochenchirurgische Haltevorrichtung**
Fixator for bone surgery
Fixateur pour chirurgie osseuse

(30) Priorität: 28.04.1994 DE 4414781; 19.07.1994 DE 4425392
(43) Veröffentlichungstag der Anmeldung: 02.11.1995
(73) Patentinhaber: SCHÄFER micomed GmbH, 73035 Göppingen (DE)
(72) Erfinder: Schäfer, Bernd, D-73614 Schorndorf (DE); Zielke, Klaus, D-34537 Bad Wildungen (DE); Rehder, Günther, D-73650 Winterbach (DE); Henry Halm, 49143 Bissendorf-Wissingen (DE)
(74) Vertreter: Dreiss, Fuhlendorf, Steimle & Becker, Patentanwälte

(56) Entgegenhaltungen:
- DE-U- 9 314 294
- US-A- 4 289 123

## Beschreibung

Die Erfindung betrifft eine knochenchirurgische Haltevorrichtung für einen Fixierstab, mit einer am Knochen befestigbaren Knochenplatte, einer Aufnahmevorrichtung für den Fixierstab und einer den Fixierstab in der Aufnahmevorrichtung haltenden Fixiereinrichtung.

Aus der EP-A-443 894 ist eine Knochenplatte bekannt geworden, die über zwei Knochenschrauben z.B. auf einem Wirbel befestigbar ist. Eine der Knochenschrauben weist einen Gabelkopf auf, in den ein Stab einlegbar und fixierbar ist. Über einen weiteren Stab können die Knochenplatten direkt miteinander verbunden werden. Eine Korrektur derart ausgebildeter Knochenplatten ist nicht oder nur sehr schwer möglich, da keine Vorkehrungen getroffen sind, einen Korrekturstab zu befestigen. Außerdem müssen die Haltevorrichtungen nach der Fixierung mit Pleura abgedeckt werden.

Es hat sich herausgestellt, daß aufgrund des relativ hohen Aufbaus dieser bekannten Haltevorrichtung unter Umständen eine Abdeckung durch Pleura nicht oder nur sehr schwierig möglich ist. Vielfach ist für die Abdeckung künstliche Pleura erforderlich, die jedoch nicht überall verfügbar und außerdem sehr kostspielig ist.

Mit der US-A-4,289,123 ist eine weitere Haltevorrichtung bekannt geworden, die auf Wirbel aufsetzbar ist. Diese Haltevorrichtung dient in der Regel zur Überbrückung einzelner Wirbel, indem auf den Nachbarwirbeln Knochenplatten aufgesetzt werden, die über zwei Gewindestangen miteinander verbunden werden. Aus biomechanischer Sicht ist eine derartige Haltevorrichtung äußerst instabil und aufwendig zu befestigen und einzurichten.

Der Erfindung liegt somit die Aufgabe zugrunde, eine chirurgische Haltevorrichtung der eingangs genannten Art derart weiterzubilden, daß sie eine höhere Stabilität und eine geringere Bauhöhe gegenüber bekannten Haltevorrichtungen aufweist.

Diese Aufgabe wird erfindungsgemäß dadurch gelöst, daß die Knochenplatte eine der Knochenoberfläche im wesentlichen angepaßte Anlagefläche aufweist und daß die Aufnahmevorrichtung einstückig mit der Knochenplatte ausgebildet ist, wobei die Aufnahmevorrichtung und die Fixiereinrichtung ein zweigeteiltes Lager bilden, so daß der Fixierstab nach der Befestigung der Knochenplatte am Knochen in die Aufnahmevorrichtung einlegbar und darin fixierbar ist.

Durch die an den Knochen bzw. an den Wirbel angepaßte Form der Knochenplatte kann diese problemlos am Wirbel befestigt werden, ohne daß dieser vorbereitet werden muß. Ist die Knochenplatte am Knochen befestigt und sind die einzelnen miteinander zu verbindenden Knochen bzw. Wirbel ausgerichtet und positioniert, dann kann das System auf einfache Weise dadurch fixiert werden, daß in die Aufnahmevorrichtung ein Fixierstab eingelegt wird. Dieser Fixierstab muß nicht, wie beim Stand der Technik seitlich eingeschoben bzw. eingeschraubt werden, was in den meisten Fällen aufgrund der beengten Verhältnisse nicht möglich ist, sondern kann in die hierfür vorgesehenen Aufnahmevorrichtungen eingelegt werden. In diesen Aufnahmevorrichtungen wird der Fixierstab fixiert und verbindet die einzelnen Knochenplatten untereinander. Das System ist primärstabil.

Erfindungsgemäß ist die Aufnahmevorrichtung einstückig mit der Knochenplatte ausgebildet. Diese Ausgestaltung hat den Vorteil, daß die Kräfte des Fixierstabes direkt auf die Knochenplatte und von dieser in den Knochen eingeleitet werden. Die Stabilität des Systems wird wesentlich erhöht.

Bei einer Weiterbildung ist vorgesehen, daß die Aufnahmevorrichtung gabelförmig ausgebildet ist und zwei distal abstehende Schenkel aufweist. Diese distal abstehenden Schenkel umgreifen den Fixierstab seitlich und halten diesen sicher an der Knochenplatte fest. Ein Verrutschen des Korrekturstabs seitlich zu dessen Längsachse ist ausgeschlossen.

Ein einfaches Einlegen des Korrekturstabs nach der Befestigung der Knochenplatte am Knochen und Positionierung der einzelnen Knochen zueinander wird dadurch erzielt, daß die Aufnahmevorrichtung nach distal offen ist.

Bei bekannten Haltevorrichtungen wird die Knochenplatte auf den Knochen befestigt und es werden die einzelnen Knochen über einen Korrekturstab in die gewünschte Lage gebracht. Sind die einzelnen Knochen positioniert, dann werden die Knochenplatten über einen Fixierstab miteinander verbunden, wodurch die Lage der einzelnen Knochen fixiert wird. Sowohl der Korrekturstab als auch der Fixierstab verbleiben im Körper des Patienten.

Bei der erfindungsgemäßen Haltevorrichtung ist vorgesehen, daß sie mit einer Werkzeugangriffseinrichtung versehen ist, an der ein Werkzeug zur Positionierung der Knochenplatte ansetzbar ist. Es wird also nicht, wie beim Stand der Technik, ein Korrekturstab angeschraubt und über diesen die Position der Knochenplatte und somit die Position des Knochens eingestellt, sondern es wird ein hierfür vorgesehenes Werkzeug an einer Werkzeugangriffseinrichtung angesetzt. Über dieses Werkzeug werden die einzelnen Knochenplatten zueinander ausgerichtet.

Vorteilhaft wird die Werkzeugangriffseinrichtung von zwei parallelen Flächen gebildet, welche an der Aufnahmevorrichtung vorgesehen sind. Auf diese parallelen Flächen ist ein gabelförmiges Werkzeug aufschiebbar und nach der Lagekorrektur der Platte wieder abziehbar. Durch die erfindungsgemäße Ausgestaltung der Haltevorrichtung mit einer Werkzeugangriffseinrichtung kann also ein Werkzeug zur Lagekorrektur der einzelnen Knochenplatten aufgeschoben und nach erfolgter Korrektur wieder abgenommen werden. Im Körper des Patienten verbleiben also keine für die Korrektur der Lage der einzelnen Wirbel erforderlichen Elemente sondern lediglich die Bauteile, die zur Fixierung benötigt werden. Dies hat den wesentlichen Vorteil, daß die Anzahl der im Körper verbleibenden Fremdkörper geringer ist und daß aufgrund der geringeren Anzahl von im Körper verbleibenden Bauteilen bzw. von am Knochen sich befindenden Bauteilen die Abdeckung durch Pleura wesentlich einfacher ist. In der Regel wird keine künstliche Pleura für die Abdeckung mehr benötigt. Außerdem wird die Bauhöhe der gesamten Haltevorrichtung beträchtlich verringert.

Bei einer Weiterbildung ist vorgesehen, daß jeder Schenkel der Aufnahmevorrichtung von zwei Fingern gebildet wird, die als Führung für eine Fixierplatte dienen. Mit dieser Fixierplatte wird der Fixierstab an der Knochenplatte fixiert.

Vorteilhaft besteht zwischen den Fingern ein Freiraum, in dem die Fixierplatte in Richtung auf die Knochenplatte beweglich geführt ist. Auf diese Weise kann eine einfache, jedoch wirkungsvolle Klemmung des Fixierstabes an der Knochenplatte herbeigeführt werden. Außerdem kann auf diese Weise die Fixierplatte selbst nach dem Einlegen des Fixierstabes auf diesen aufgesetzt und an der Knochenplatte befestigt werden.

Nach dem Aufsetzen wird ein Verschieben der Fixierplatte parallel zur Knochenplatte dadurch verhindert, daß die Finger der Knochenplatte die Fixierplatte in horizontaler Ebene festhalten. Die Platte ist dann lediglich orthogonal zur Knochenplatte verschiebbar.

Bevorzugt ist die Fixierplatte über eine oder mehrere Befestigungseinrichtungen an der Knochenplatte bzw. auf dem Fixierstab befestigbar. Dies erfolgt dann, wenn die Knochenplatte die gewünschte Position einnimmt und nachdem der Fixierstab eingelegt worden ist. Mit der Befestigung der Fixierplatte auf der Knochenplatte wird diese stabil mit dem Fixierstab verbunden, wodurch die Primärstabilität für die einzelnen Wirbel erzielt wird.

Die Befestigungseinrichtungen werden von zwei Schrauben gebildet, die unverlierbar an der Fixierplatte befestigt sind. Dies hat den Vorteil, daß unmittelbar nach dem Aufsetzen der Fixierplatte diese mit der Knochenschraube verbunden werden kann.

Die Schrauben sind entlang der Längsachse der Fixierplatte angeordnet, so daß eine optimale Übertragung der Kräfte vom Fixierstab auf die Knochenplatte erreicht wird.

Eine Verdrehung des Fixierstabes in der Aufnahmevorrichtung wird dadurch verhindert, daß die Fixierplatte an ihrer dem Fixierstab zugewandten Seite wenigstens abschnittsweise eine Profilierung aufweist. Der Grund der Aufnahmevorrichtung kann ebenfalls mit einer Profilierung versehen sein. Bei längsgenuteten Fixierstäben weist diese Profilierung komplementäre Längsnuten auf.

Bei einem bevorzugten Ausführungsbeispiel ist die Aufnahmevorrichtung zur Aufnahme eines insbesondere längsgenuteten Korrekturstabes mit 6 mm Durchmesser ausgebildet. Dabei weist die Haltevorrichtung eine Höhe von etwa 8 mm bis 10 mm auf.

Die Aufnahmevorrichtung ist zwischen zwei Befestigungsbohrungen, über die die Knochenplatte am Knochen befestigbar ist, vorgesehen. Diese Lage hat den Vorteil, daß die vom Fixierstab auf die Knochenplatte eingeleiteten Kräfte im wesentlichen gleichmäßig auf die Knochenschrauben und somit in den Knochen eingeleitet werden. Eine dezentrale Anordnung der Aufnahmevorrichtung ist ebenfalls möglich.

Eine spielfreie Befestigung der Knochenplatte am Knochen wird dadurch erzielt, daß jede Befestigungsbohrung einen konischen Sitz für die Knochenschrauben aufweist.

Bevorzugt ist die Knochenplatte spiegelsymmetrisch aufgebaut, wodurch eine optimale Kräfteverteilung erzielt wird.

Die erfindungsgemäße Haltevorrichtung wird unter anderem bei Tumoren, Frakturen, Kyphosen, Skoliosen und Spondylolisthesis verwendet.

Bei einer Weiterbildung des erfindungsgemäßen Gegenstandes ist vorgesehen, daß die Knochenplatte eine Verbindungseinrichtung für ein zum Beispiel wenigstens einen Korrekturstab enthaltendes, nach der Korrektur und Fixierung entfernbares Korrekturinstrument aufweist.

Durch die erfindungsgemäße knochenchirurgische Haltevorrichtung wird die Möglichkeit geschaffen, daß für die Lagekorrektur des Wirbels nunmehr ein Instrument verwendet werden kann, welches nach der Fixierung der Knochenplatte wieder entfernbar ist. Bei der erfindungsgemäßen knochenchirurgischen Haltevorrichtung wird also die Knochenplatte am zu korrigierenden Knochen, z.B. an einem Wirbel einer Wirbelsäule befestigt und mittels der Verbindungseinrichtung wird das Korrekturinstrument an der Knochenplatte befestigt, so daß über das Korrekturinstrument die Lage der Knochenplatte und somit die Lage des Knochens, z.B. des Wirbels, korrigiert werden kann. Dabei spielt die Größe des Korrekturinstruments bzw. z.B. eines in das Instrument einlegbaren Korrekturstabes nur eine untergeordnete Rolle, da das Instrument mit Korrekturstab nach der Fixierung wieder entfernt wird. Nimmt die Knochenplatte bzw. der Wirbel die gewünschte Lage ein, dann wird die Lage der Knochenplatte, z.B. mittels eines Fixierstabes, fixiert. Nach der Fixierung wird das Korrekturinstrument entfernt und die Operation kann abgeschlossen werden. Im Körper des Patienten verbleiben demnach lediglich die Knochenplatte sowie die für die Fixierung der Knochenplatte erforderlichen Bestandteile der Haltevorrichtung.

Neben einem geringerem Bauvolumen weist die erfindungsgemäße knochenchirurgische Haltevorrichtung eine geringere Höhe, geringere Breite und geringere Länge als herkömmliche Haltevorrichtungen auf, so daß sie allgemein verträglicher ist. Aufgrund des geringeren Bauvolumens, insbesondere der geringeren Höhe kann die Haltevorrichtung einfacher mit Pleura bedeckt werden. Außerdem besitzt die an den Knochen bzw. an den Wirbel angepaßte Form der Knochenplatte den Vorteil, daß die Befestigung problemlos durchzuführen ist, ohne daß der Knochen vorbereitet werden muß. Durch die Befestigung des Fixierstabes direkt an der Knochenplatte ist das System primärstabil.

Durch die mehrfache Verwendung des Korrekturinstruments werden die Kosten für Operationen gesenkt und die im Körper des Patienten verbleibende Haltevorrichtung baut relativ klein, wobei das ebenfalls geringere Gewicht nicht unerwähnt bleiben soll.

Bei einer bevorzugten Ausführungsform ist das Korrekturinstrument über die Verbindungseinrichtung einschiebbar. Nach der Befestigung der Haltevorrichtung am Knochen kann das Korrekturinstrument auf einfache Weise durch Aufschieben bzw. Einschieben mit diesem verbunden werden. Zeitaufwendige Verschraubungen sind nicht erforderlich. Dabei entspricht die Einschubrichtung des Korrekturinstruments im wesentlichen der Längsrichtung der Knochenplatte. Bei anderen Ausführungsformen entspricht die Einschubrichtung der Querrichtung oder liegt diagonal zu dieser. Bevorzugt entspricht die Einschubrichtung der Längsrichtung der Aufnahmevorrichtung für den Fixierstab.

Um eine definierte Lage des Korrekturinstruments an der Knochenplatte zu erzielen, weist das Korrekturinstrument einen als Einschubbegrenzer dienenden Anschlag auf. Dieser Anschlag erlaubt ein Einschieben bzw. ein Aufschieben des Korrekturinstruments auf die erfindungsgemäße Haltevorrichtung so weit, bis der Anschlag an der Haltevorrichtung anliegt. Das Korrekturinstrument nimmt nun die vorbestimmte Lage ein, und die Korrekturkräfte können optimal in die Haltevorrichtung eingeleitet werden.

Gemäß einem Ausführungsbeispiel wird die Verbindungseinrichtung von einem ersten, am Korrekturinstrument vorgesehenen Teil und einem zweiten, an der Knochenplatte vorgesehenen Teil gebildet, wobei die beiden Teile zum Beispiel eine Nut-Feder-Verbindung oder eine Loch-Zapfen-Verbindung bilden. Derart ausgestaltete Verbindungseinrichtungen gewährleisten einen optimalen Halt des Korrekturinstruments an der Knochenplatte, so daß ein versehentliches Abgleiten bzw. Abrutschen des Korrekturinstruments nicht zu befürchten ist.

Bei einer anderen Ausführungsform ist vorgesehen, daß die Verbindungseinrichtung an der Aufnahmevorrichtung für den Fixierstab vorgesehen ist. Hier wird die Verbindungseinrichtung z.B. von zwei parallelen Flächen gebildet. Dabei kann wenigstens eine der Flächen mit einer in Einschubrichtung verlaufenden Nut bzw. mit einem Vorsprung versehen sein, die bzw. der als Führung für das Korrekturinstrument dient. Auch hier wird durch die Ausgestaltung der Verbindungseinrichtung ein optimaler Verbund zwischen Korrekturinstrument und Aufnahmevorrichtung gewährleistet. Die Verbindungseinrichtung erlaubt eine eindimensionale Relativbewegung zwischen Korrekturinstrument und Knochenplatte. Auf diese Weise wird eine einfache Möglichkeit geschaffen, daß in allen anderen Richtungen Kräfte vom Korrekturinstrument auf die Knochenplatte übertragen werden können. Wird die Knochenplatte mit dem Korrekturinstrument verriegelt, kann auch in Einschubrichtung eine Kraft übertragen werden.

Bei einer Weiterbildung ist vorgesehen, daß das Korrekturinstrument im wesentlichen gabelförmig ausgebildet ist, wobei bei aufgeschobenem bzw. aufgesetztem Korrekturelement die Gabelschenkel die Aufnahmevorrichtung für den Fixierstab umgreifen bzw. flankieren. Ein gabelförmiges Korrekturinstrument hat den wesentlichen Vorteil, daß es auf einfache Weise die Aufnahmevorrichtung umgreifen und somit die Korrekturkräfte gezielt und sicher in dieses einleiten kann.

Bevorzugt ist in das Korrekturinstrument ein Korrekturstab einlegbar und befestigbar. Dabei kann das Korrekturinstrument sowohl mit als auch ohne Korrekturstab mit der Knochenplatte bzw. der Aufnahmevorrichtung verbunden werden, wobei bevorzugt wird, daß zunächst das Korrekturinstrument an der Knochenplatte bzw. der Aufnahmevorrichtung befestigt wird und danach der Korrekturstab in das Korrekturinstrument eingelegt und ebenfalls befestigt wird. Danach kann die Lagekorrektur der einzelnen Haltevorrichtungen über den oder die Korrekturstäbe stattfinden.

Zur Erhöhung der Primärstabilität ist eine Weiterbildung der erfindungsgemäßen knochenchirurgischen Haltevorrichtung so ausgestaltet, daß neben dem oben erwähnten Fixierstab ein weiterer Fixierstab, gegebenenfalls mit einem kleineren Durchmesser, mit der Knochenplatte verbindbar ist. Dabei liegen die beiden Fixierstäbe im wesentlichen parallel und bilden einen H-Rahmen. Die Befestigung des zweiten Fixierstabes kann z.B. direkt an der Platte erfolgen und entspricht im wesentlichen der Fixierung des oben erwähnten ersten Fixierstabes. Eine Variante sieht vor, daß anstelle einer der beiden Befestigungsschrauben, über die die Knochenplatte am Knochen befestigt wird, eine Knochenschraube mit einem Gabelkopf verwendet wird, über die sowohl die Knochenplatte am Knochen befestigt wird, in die jedoch auch der Fixierstab eingelegt und befestigt werden kann. Diese Schraube dient also zum Befestigen der erfindungsgemäßen Haltevorrichtung als auch zur Festlegung des zweiten Fixierstabes. Diese Schraube befindet sich bevorzugt auf der dorsalen Seite der Haltevorrichtung. Die Befestigung des Fixierstabes nach dem Einschieben bzw. Einlegen erfolgt z.B. über eine Hutmutter, kann jedoch auch über ein verlängertes Ende der Fixiereinrichtung bzw. der Fixierplatte erfolgen, wobei das verlängerte Ende über die Aufnahmevorrichtung absteht und den zweiten Fixierstab übergreift und diesen festhält. Diese Variante mit einem zusätzlichen Fixierstab kann bei allen Ausführungsformen der erfindungsgemäßen knochenchirurgischen Haltevorrichtung verwirklicht sein.

Bevorzugt weist die Aufnahmevorrichtung einen im wesentlichen rechteckförmigen Grundriß auf. An eine derart ausgestaltete Aufnahmevorrichtung kann auf einfache Weise, z.B. durch Aufsetzen oder Aufschieben ein Korrekturinstrument angelegt und an dieser befestigt werden.

Bevorzugt weist die Längsachse der Aufnahmevorrichtung zur Längsachse der Knochenplatte einen von 0° verschiedenen Winkel auf. Außerdem kann die Längsachse der Knochenplatte zur Längsachse des in die Aufnahmevorrichtung einsetzbaren Fixierstabes einen von 90° abweichenden Winkel aufweisen.

Zur Verringerung der Gesamthöhe der Haltevorrichtung liegen der Grund der Aufnahmevorrichtung für den Fixierstab und die zum Knochen distale Oberfläche der Knochenplatte, wenigstens abschnittsweise, im wesentlichen in der gleichen Ebene. Der Fixierstab ist also lediglich über die Knochenplatte von der Oberfläche des Knochens beabstandet.

Bei einer Weiterbildung ist vorgesehen, daß die Fixiereinrichtung für den Fixierstab wenigstens ein Lager für wenigstens einen Haken der Aufnahmevorrichtung aufweist und daß die Fixiereinrichtung an der Aufnahmevorrichtung einhängbar ist. Zur Befestigung der Fixiereinrichtung für den Fixierstab an der Aufnahmevorrichtung ist diese an der Aufnahmevorrichtung anschraubbar. Bevorzugt weist die Fixiereinrichtung wenigstens eine unverlierbare Schraube auf.

Bei einer Weiterbildung ist vorgesehen, daß die Fixiereinrichtung als einhängbarer Klemmdeckel ausgebildet ist. Die Einhängvorrichtung besitzt den Vorteil, daß die Fixiereinrichtung einfach, problemlos und schnell mit der Aufnahmevorrichtung verbindbar und z.B. über eine einzige Schraube an dieser befestigbar ist, wobei über die Schraube der Fixierstab eingeklemmt wird. Da lediglich eine einzige Schraube bedient werden muß, kann der Fixierstab schneller und problemloser befestigt werden als bei herkömmlichen Haltevorrichtungen, was insbesondere die Operationszeit verkürzt.

Unverlierbare Schrauben sind ebenfalls zu bevorzugen, da mit diesen die Operationsrisiken weiter gemindert werden.

Bei einem bevorzugten Ausführungsbeispiel weist die Knochenplatte im wesentlichen diagonal angeordnete Aufnahmen für Befestigungsschrauben auf. Durch die diagonale Anordnung wird die Gesamtlänge der Haltevorrichtung vermindert, wobei die Längsachse der Aufnahmevorrichtung in einer dazu komplementär verlaufenden Diagonalen liegt, was deutlich aus der Zeichnung erkennbar ist.

Weitere Vorteile, Merkmale und Einzelheiten der Erfindung ergeben sich aus der nachfolgenden Beschreibung, in der unter Bezugnahme auf die Zeichnung besonders bevorzugte Ausführungsbeispiele im einzelnen beschrieben sind. Dabei können die in der Zeichnung dargestellten und in den Ansprüchen und in der Beschreibung erwähnten Merkmale jeweils einzeln für sich oder in beliebiger Kombination erfindungswesentlich sein. In der Zeichnung zeigen:
- Figur 1: eine Seitenansicht einer ersten Ausführungsform einer Knochenplatte der erfindungsgemäßen Haltevorrichtung;
- Figur 2: einen Längsschnitt durch eine Fixierplatte;
- Figur 3: Schrauben zur Befestigung der Fixierplatte auf der Knochenplatte;
- Figur 4: eine Seitenansicht der erfindungsgemäßen Haltevorrichtung mit auf der Knochenplatte aufgeschraubter Fixierplatte und mit in die Knochenplatte eingesetzten Knochenschrauben;
- Figur 5: eine Draufsicht auf die Haltevorrichtung gemäß den Figuren 1 bis 3;
- Figur 6: eine Seitenansicht einer zweiten Ausführungsform einer Knochenplatte der erfindungsgemäßen Erfindung;
- Figur 7: eine Seitenansicht der Fixiereinrichtung;
- Figur 8: einen Längsschnitt VIII-VIII gemäß Figur 9 durch die Knochenplatte mit aufgesetzter Fixiereinrichtung;
- Figur 9: eine Draufsicht auf die Knochenplatte mit Fixiereinrichtung;
- Figur 10: eine Draufsicht auf ein abgenommenes Korrekturinstrument;
- Figur 11: eine Seitenansicht des Korrekturinstruments gemäß Figur 10;
- Figur 12: eine Ansicht in Richtung des Pfeils XII gemäß Fig. 10.

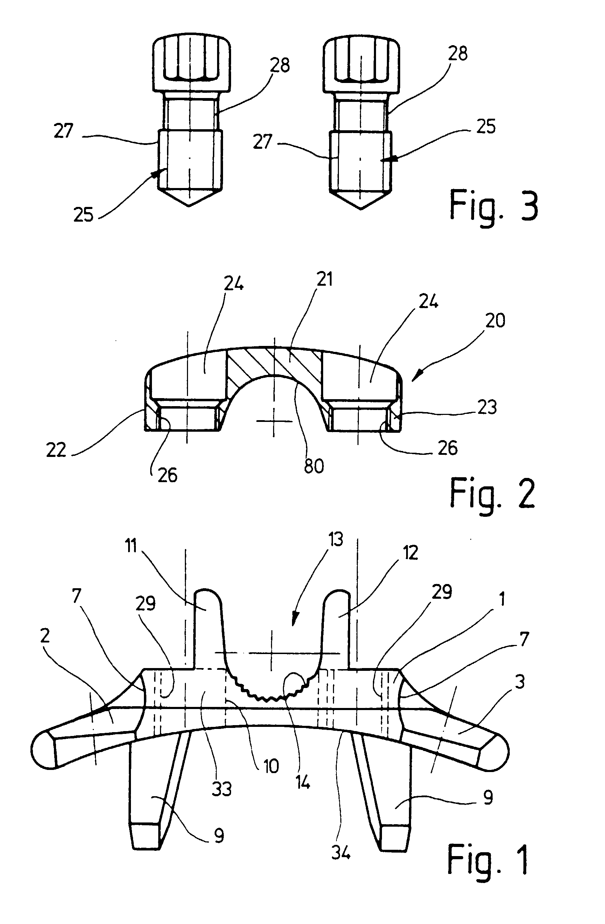

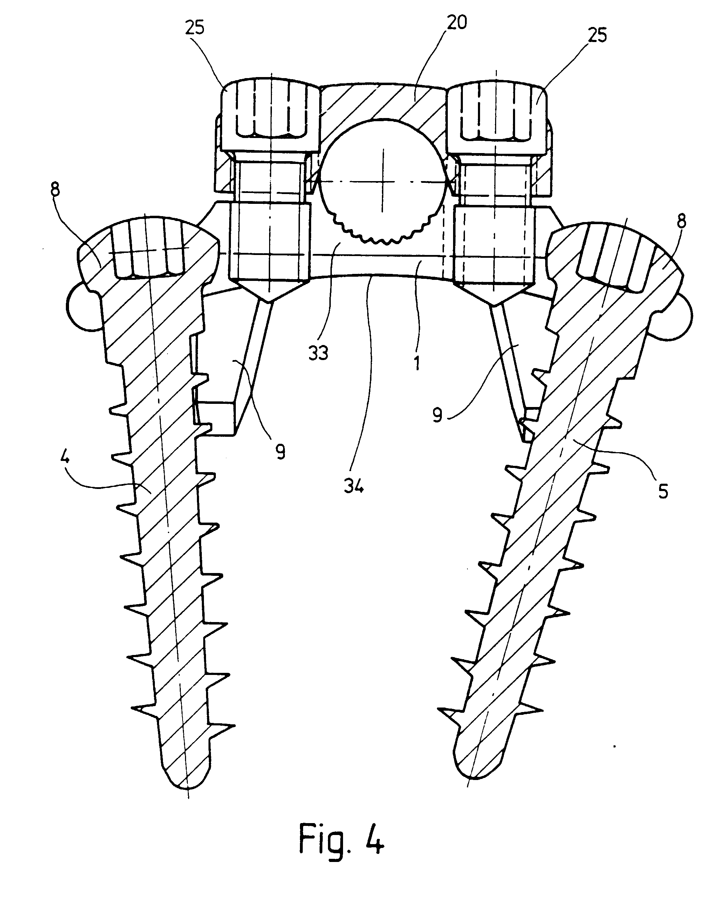

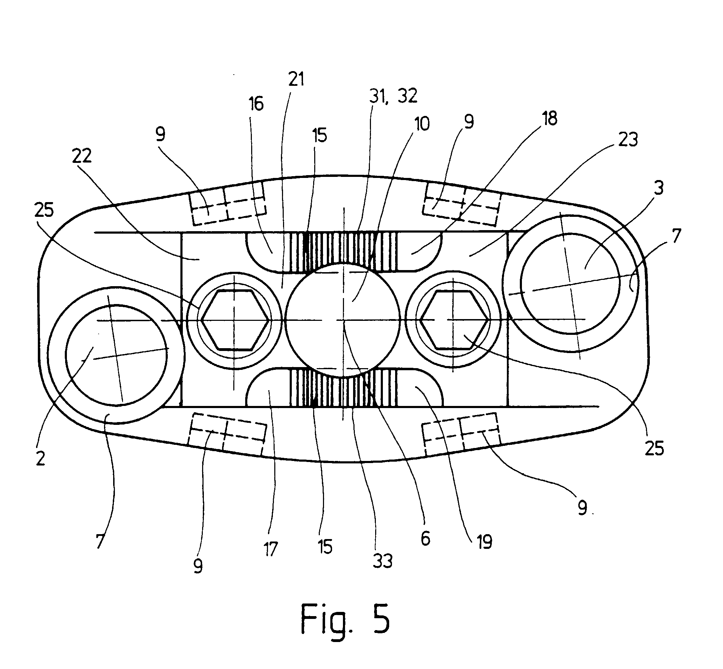

Die Figur 1 zeigt eine Knochenplatte 1 eines ersten Ausführungsbeispiels in Seitenansicht, wobei zwei Befestigungsbohrungen 2 und 3 erkennbar sind, über die die Knochenplatte 1 an einem nicht dargestellten Knochen über geeignete Knochenschrauben 4 und 5 (Figur 4) befestigbar ist. Bevorzugt sind die beiden Befestigungsbohrungen 2 und 3 bezüglich des Zentrums 6 (Figur 5) der Knochenplatte 1 einander gegenüberliegend angeordnet. Die Befestigungsbohrungen 2 und 3 weisen jeweils ein kalottenförmiges Lager 7 auf, was jedoch auch konisch ausgestaltet sein kann. Von diesem kalottenförmigen Lager 7 wird der Kopf 8 der Knochenschraube 4 bzw. 5 aufgenommen, der entsprechend sphärisch ausgebildet ist. Das Lager 7 hat den Vorteil, daß die Knochenschraube 4 bzw. 5 nicht zwingend koaxial zur Befestigungsbohrung 2 bzw. 3 ausgerichtet sein muß, sondern geringe Schrägstellungen einnehmen kann, so daß die Knochenschraube 4 bzw. 5 in geeignete Bereiche des Knochens eingeschraubt werden kann.

Ferner ist aus den Figuren 1 und 4 erkennbar, daß die Befestigungsbohrung 2 schräggestellt ist, wobei die Achse der Befestigungsbohrung 3 orthogonal zur Knochenplatte 1 und die Achse der Befestigungsbohrung 2 im wesentlichen parallel zu Ankerstiften 9 verläuft.

In Figur 1 ist außerdem erkennbar, daß die Knochenplatte 1 mit einem zentralen Durchbruch 10 versehen ist, der bei dem in der Figur 1 dargestellten Ausführungsbeispiel kreisförmig ist.

An der Oberseite der Knochenplatte 1 sind zwei gabelförmige Schenkel 11 und 12 vorgesehen, die eine Aufnahmevorrichtung 13 für einen nicht dargestellten Fixierstab bilden. Dieser Fixierstab kann zwischen die beiden Schenkel 11 und 12 in die Aufnahmevorrichtung 13 eingelegt werden und liegt am Grund 14 auf. Dieser Grund 14 ist, wie auch deutlich aus Figur 5 erkennbar, mit Längsnuten 15 versehen, die in komplementäre Längsnuten des Fixierstabes eingreifen. Dies Längsnuten 15 sind hinterschneidungsfrei ausgebildet, so daß der Fixierstab von oben problemlos in die Aufnahmevorrichtung 13 eingesetzt werden kann. Die Längsnuten 15 verhindern ein Verdrehen des Fixierstabes in der Aufnahmevorrichtung 13. Ein problemloses Einsetzen des Fixierstabes zwischen die beiden Schenkel 11 und 12 wird dadurch erleichtert, daß diese eine im wesentlichen konische Einschuböffnung bilden.

Aus Figur 5 ist erkennbar, daß die beiden Schenkel 11 und 12 von jeweils zwei Fingern 16 bis 19 gebildet werden. Diese Finger 16 bis 19 dienen als Führung für eine Fixierplatte 20, welche in der Figur 2 dargestellt ist. Die Fixierplatte 20 weist in der Draufsicht eine H-förmige Gestalt auf, was sich aus Figur 5 ergibt. Wird die Fixierplatte 20 auf die Knochenplatte 1 aufgesetzt, dann durchgreift ein Steg 21 den zwischen den beiden Fingern 16 und 17 bzw. 18 und 19 gebildeten Freiraum und überbrückt im wesentlichen den Auflagebereich für den Fixierstab. Dieser Steg 21 verbreitert sich an seinen Enden derart, daß er die Finger 16 bis 19 an zweiten Seiten umgreift. In den beiden verbreiterten Enden 22 und 23 befinden sich außerdem Bohrungen 24 zur Aufnahme von Schrauben 25, welche in Figur 3 dargestellt sind.

Diese Bohrungen 24 sind an ihrem unteren Ende mit einem Gewinde 26 versehen, wodurch eine unverlierbare Halterung für die Schrauben 25 geschaffen wird. Die beiden Schrauben 25 weisen ein Gewinde 27 auf, welches im oberen Bereich 28 abgedreht ist. Befindet sich die Schraube 25 derart in der Bohrung 24, daß der Bereich 28 im Bereich des Gewindes 26 zum Liegen kommt, kann die Schraube 25 frei gedreht werden. Ein Einschrauben des Gewindes 27 in ein Gewinde 29 in der Knochenplatte 1 ist dann möglich, so daß die Fixierplatte 20 auf einen Fixierstab aufklemmbar ist.

Die Unterseite 30 der Fixierplatte 20 kann im Bereich zwischen den beiden Bohrungen 24 ebenfalls mit Längsnuten versehen sein. Hierdurch wird eine Verdrehung des Fixierstabes um seine Längsachse effektiv vermieden.

In der Figur 4 ist eine komplette Haltevorrichtung dargestellt, bei der die Fixierplatte 20 mittels der Schrauben 25 auf die Knochenplatte 1 aufgeschraubt ist. Es ist deutlich erkennbar, daß dadurch, daß der Fixierstab direkt in die Knochenplatte 1 eingelegt wird, die Fixierkräfte direkt auf die Knochenplatte 1 und von dieser über die Ankerstifte 9 und die Knochenschrauben 4 und 5 in den Knochen eingeleitet werden können. Außerdem wird eine sehr geringe Bauhöhe erzielt, da der Fixierstab unmittelbar oberhalb der Knochenoberfläche liegt. Eine Abdeckung dieser Haltevorrichtung durch Pleura ist problemlos möglich.

Ein weiterer erfindungswesentlicher Gesichtspunkt wird darin gesehen, daß die Aufnahmevorrichtung 13 eine Werkzeugangriffseinrichtung 31 in Form von zwei zueinander parallelen Flächen 32 und 33 aufweist.Die Werkzeugangriffseinrichtung 31 weist einen Abstand zur Knochenoberfläche auf, so daß dieser durch das angreifende Werkzeug nicht verletzt wird. Diese Flächen 32 und 33 erlauben einem Werkzeug, z.B. einer entsprechend geformten Gabel oder einer Klammer ein müheloses Ergreifen der Knochenplatte 1. Ist die Knochenplatte 1 am Knochen befestigt, dann wird die Knochenplatte 1 und mit dieser der Knochen über das Werkzeug mittels eines Korrekturstabs in die gewünschte Position gebracht. Nach der Ausrichtung der Knochenplatten 1 werden diese über den Fixierstab, der in die jeweiligen Aufnahmevorrichtungen 13 eingelegt und dort befestigt wird, in der vorgegebenen Position fixiert. Danach können der Korrekturstab und die einzelnen Werkzeuge zum Ausrichten der Knochenplatten 1 von der Werkzeugangriffseinrichtung 31 abgenommen werden. Eine Beibehaltung der Position der Knochenplatten 1 wird durch den Fixierstab gewährleistet. Dieser weist zur sicheren Aufnahme der auftretenden Kräfte einen geringfügig größeren Durchmesser auf, als bei Einrichtungen, bei denen sowohl ein Korrekturstab als auch ein Fixierstab implantiert werden.

Aus den Figuren 1 und 4 ist außerdem deutlich erkennbar, daß die Anlagefläche 34 der Knochenplatte 1 eine im wesentlichen gleichmäßig verlaufende konkave Krümmung aufweist, die in etwa der Krümmung der Knochenoberfläche entspricht. Hierdurch wird ein sattes Anliegen der Knochenplatte 1 auf der Knochenoberfläche gewährleistet, so daß die Kräfte der Knochenplatte 1 flächig in den Knochen eingeleitet werden können.

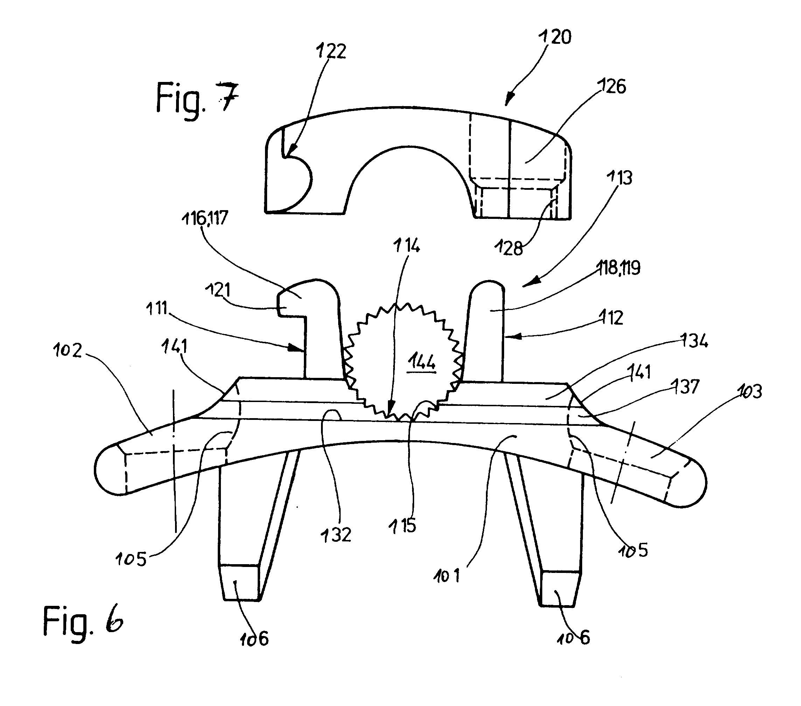

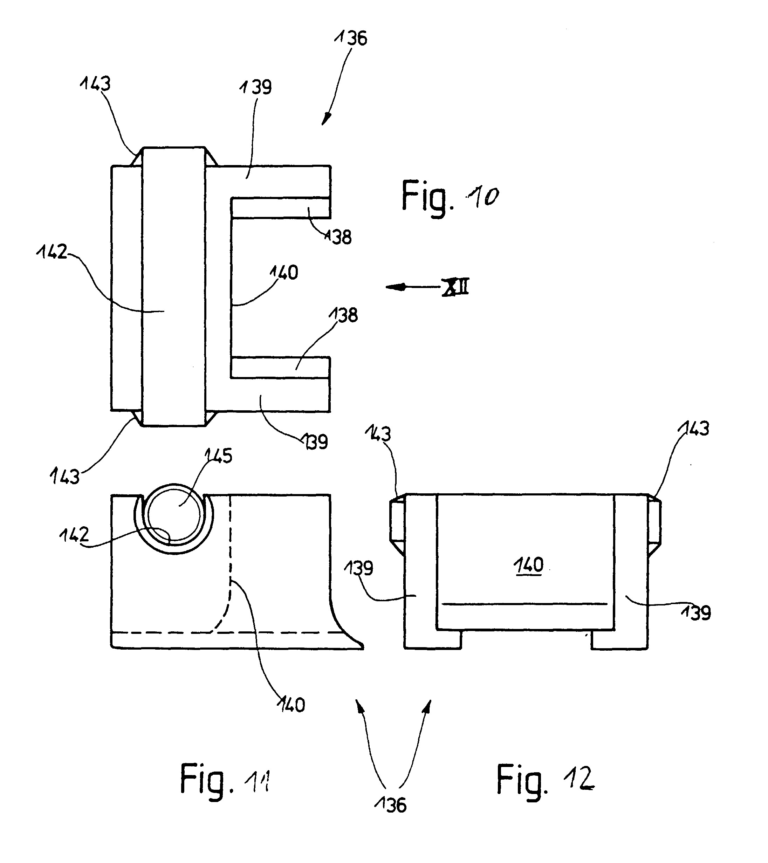

Die Figur 6 zeigt eine Knochenplatte 101 in Seitenansicht, wobei zwei Befestigungsbohrungen 102 und 103 mit gestrichelten Linien dargestellt sind, über die die Knochenplatte 101 an einem nicht dargestellten Knochen über geeignete, ebenfalls nicht dargestellte Knochenschrauben befestigbar ist. Bevorzugt sind die beiden Befestigungsbohrungen 102 und 103 bezüglich des Zentrums 104 (siehe Figur 9) der Knochenplatte 101 einander gegenüberliegend angeordnet. Die Befestigungsbohrungen 102 und 103 weisen jeweils ein kalottenförmiges Lager 105 auf, was jedoch auch konisch ausgestaltet sein kann. Von diesem kalottenförmigen Lager 105 wird der Kopf der Knochenschraube aufgenommen, der entsprechend sphärisch ausgebildet. Das Lager 105 hat den Vorteil, daß die Knochenschraube nicht zwingend koaxial zur Befestigungsbohrung 102 bzw. 103 ausgerichtet sein muß, sondern geringe Schrägstellungen einnehmen kann, so daß die Knochenschraube in geeignete Bereiche des Knochens eingeschraubt werden kann.

Ferner ist aus der Figur 6 erkennbar, daß die Befestigungsbohrung 102 schräggestellt ist, wobei die Achse der Befestigungsbohrung 103 orthogonal zur Knochenplatte 101 bzw. radial zu deren Krümmung und die Achse der Befestigungsbohrung 102 im wesentlichen parallel zu Ankerstiften 106 verläuft. Aus Figur 9 ist außerdem erkennbar, daß die beiden Befestigungsbohrungen 102 und 103 auf einer Diagonalen liegen, die bezüglich der Längsachse 107 der Knochenplatte 101 entgegen dem Uhrzeigersinn geneigt ist. Schließlich ist die Knochenplatte 101, was ebenfalls aus Figur 9 erkennbar ist, mit einem zentralen Durchbruch 108 versehen, der im wesentlichen kreisförmig ist.

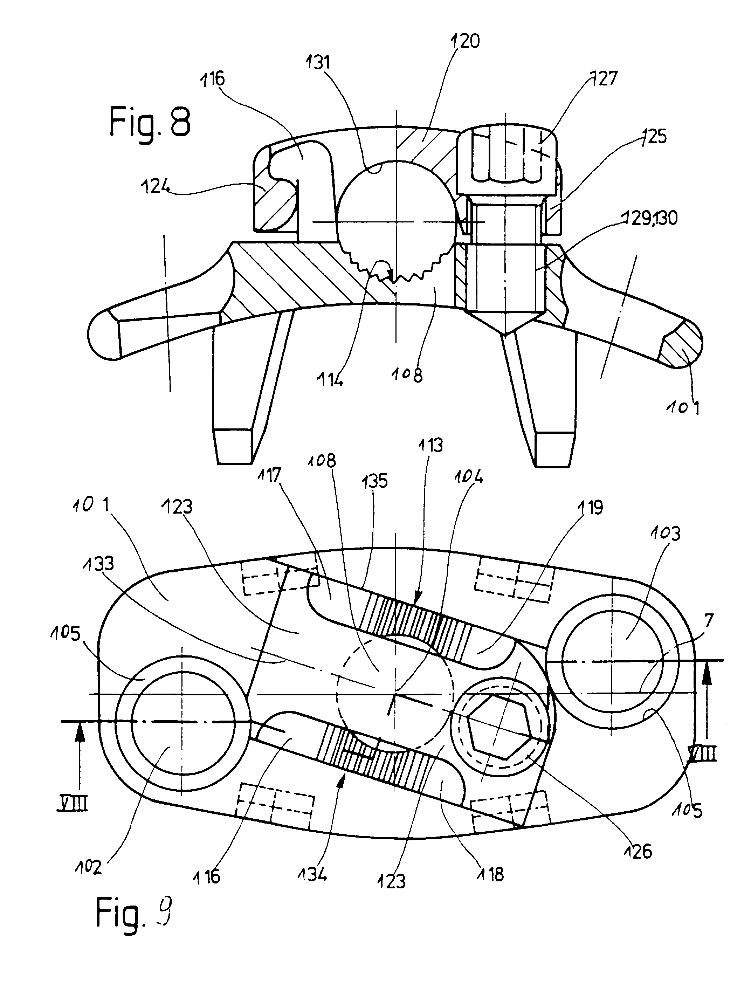

An der Oberseite der Knochenplatte 101 sind zwei gabelförmige Schenkel 111 und 112 vorgesehen, die eine Aufnahmevorrichtung 113 für einen nicht dargestellten Fixierstab bilden. Dieser Fixierstab kann zwischen die beiden Schenkel 111 und 112 in die Aufnahmevorrichtung 113 eingelegt werden und liegt am Grund 114 auf. Dieser Grund 114 ist, wie deutlich aus den Figuren 6 und 8 erkennbar, mit Längsnuten 115 versehen, die in komplementäre Längsnuten des Fixierstabes eingreifen. Diese Längsnuten 115 sind hinterschneidungsfrei ausgebildet, so daß der Fixierstab von oben problemlos in die Aufnahmevorrichtung 113 eingesetzt werden kann. Die Längsnuten 115 verhindern ein Verdrehen des Fixierstabes in der Aufnahmevorrichtung 113. Ein problemloses Einsetzen des Fixierstabes zwischen die beiden Schenkel 111 und 112 wird außerdem dadurch erleichtert, daß diese eine im wesentlichen konische Einschuböffnung bilden.

Aus den Figuren 6 und 9 ist erkennbar, daß die beiden Schenkel 111 und 112 von jeweils zwei Fingern 116 bis 119 gebildet werden. Diese Finger 116 bis 119 dienen sowohl als Führung als auch als Halterung für eine Fixiereinrichtung 120 bzw. eine Fixierplatte, die in Figur 7 dargestellt ist. Die Fixierplatte 120 weist in der Draufsicht eine H-förmige Gestalt auf, was sich aus Figur 9 ergibt. Um die Fixierplatte 120 in die Aufnahmevorrichtung 113 einhängen zu können, sind die beiden Finger 116 und 117 hakenförmig ausgebildet und weisen an ihrem distalen freien Ende einen radial nach außen abstehenden Vorsprung 121 auf. Zur Aufnahme dieses Vorsprungs 121 ist die Fixierplatte 120 an den freien Enden eines ihrer H-Schenkels 124 mit jeweils einer Hinterschneidung 122 versehen, in die der Vorsprung 121 eingehängt werden kann, wie es in der Figur 8 dargestellt ist. Wird die Fixierplatte 120 in die Finger 116 und 117 eingehängt und über das von den Vorsprüngen 121 und den Hinterschneidungen 122 gebildete Gelenk in Richtung des Uhrzeigersinns auf die Knochenplatte 101 aufgesetzt, dann durchgreift ein Steg 123 den zwischen den beiden Fingern 116 und 117 sowie 118 und 119 gebildeten Freiraum und überbrückt im wesentlichen den Auflagebereich für den Fixierstab. Dieser Steg 121 verbreitert sich an seinen Enden derart, daß er die Finger 116 und 117 mit dem H-Schenkel 124 (Figur 8) und die beiden Finger 118 und 119 mit dem H-Schenkel 125 (Figur 8) umgreift bwz. hintergreift.

In dem beim H-Schenkel 125 sich befindenden Stegbereich befindet sich außerdem eine Bohrung 126, die zur Aufnahme einer Schraube 127 dient, die in Figur 8 dargestellt ist. Diese Bohrung 126 ist an ihrem unteren Ende, d.h. an ihrem der Knochenplatte 101 zugewandten Ende, mit einem Gewinde 128 versehen, wodurch eine unverlierbare Halterung für die Schraube 127 geschaffen wird. Die Schraube 127 weist ebenfalls ein Gewinde 129 auf, welches im oberen Bereich abgedreht ist. Befindet sich die Schraube 127 derart in der Bohrung 126, daß der abgedrehte Bereich im Gewinde 128 der Fixierplatte 120 Zu liegen kommt, dann kann die Schraube 127 frei gedreht werden. Ein Einschrauben der Schraube 127 in ein Gewinde 130 in der Knochenplatte 101 ist dann möglich, so daß die Fixierplatte 120 auf einen Fixierstab aufklemmbar ist, wobei die Fixierplatte 120 um das vom Vorsprung 121 und der Hinterschneidung 122 gebildete Gelenk in Richtung des Uhrzeigersinns verschwenkt wird.

Die Unterseite 131 der Fixierplatte 120 kann im Bereich zwischen den Fingern 116 bis 119 ebenfalls mit Längsnuten (nicht dargestellt) versehen sein. Hierdurch wird eine zusätzliche Verdrehverhinderung für den Fixierstab 144 um dessen Längsachse geschaffen.

Aus den Figuren 6 und 8 ist außerdem deutlich erkennbar, daß der Grund 114 der Aufnahmevorrichtung 113 auf der Ebene 132 der Knochenplatte 101 liegt, so daß der Fixierstab 144 mit minimalem Abstand zur Knochenoberfläche liegt und die gesamte Halteeinrichtung eine geringe Bauhöhe besitzt.

Aus Figur 9 ist erkennbar, daß die Aufnahmevorrichtung 113 derart auf der distalen Seite der Knochenplatte 101 angeordnet ist, daß die Längsachse 133 diagonal auf der distalen Oberseite der Knochenplatte 101 verläuft, d.h. in Richtung des Uhrzeigersinns gegenüber der Längsachse 107 der Knochenplatte 101 gedreht ist. Die Verdrehung der Längsachse 133 der Aufnahmevorrichtung 113 und die Diagonale, in der die beiden Befestigungsbohrungen 102 und 103 liegen, sind bezüglich der Längsachse 107 der Knochenplatte 101 in verschiedene Richtungen gedreht. Auf diese Weise wird eine kurze Baulänge der Knochenplatte 101 geschaffen.

Aus den Figuren 6 und 9 ist außerdem erkennbar, daß die Aufnahmevorrichtung 113 paralelle Seitenflächen 134 und 135 aufweist, die zur Aufnahme eines Korrekturinstruments 136 dienen, welches in den Figuren 10 bis 12 dargestellt ist. Die beiden Seitenflächen 134 und 135 sind jeweils mit einer Längsnut 137 versehen, in die ein Vorsprung 138 zweier Gabelschenkel 139 eingreifen. Die Innenflächen der Gabelschenkel 139 sind so weit voneinander beabstandet, daß sie gerade die Aufnahmevorrichtung 113 aufnehmen können. Die Wand 140 dient als Anschlag zur Einschubbegrenzung des Korrekturinstruments 136 und liegt bei aufgeschobenem Instrument bei 141 an. Außerdem weist das Korrekturinstrument 136 eine im wesentlichen U-förmige Ausnehmung 142 auf, in die ein Korrekturstab (Gewindestab) einsetzbar ist. Die Enden der Ausnehmung 142 sind mit konisch zulaufenden Vorsprüngen 143 versehen, die kreisförmig um den Rand der Ausnehmung 142 herumlaufen. Diese Vorsprünge 143 dienen zur Aufnahme einer Befestigungsmutter für den Korrekturstab, wobei die Befestigungsmutter (nicht dargestellt) eine entsprechend konische Ausnehmung besitzt. Derartige Muttern sind bekannt.

Nach dem Befestigen der Knochenplatte 101 auf der Oberfläche des zu korrigierenden Knochens, z.B. eines Wirbels, wird das Korrekturinstrument 136 auf die Aufnahmevorrichtung 113 aufgeschoben. Bei mehreren Knochenplatten 101 werden entsprechend mehrere Korrekturinstrumente 136 aufgeschoben. In die einzelnen Korrekturinstrumente 136 wird nun ein Korrekturstab 145 eingelegt und über die Muttern an den Vorsprüngen 143 befestigt. Durch Verdrehen der Muttern kann die Lage des Korrekturinstruments 136 bezüglich des Korrekturstabes 145 und somit die Lage der Knochenplatte 101 und mit dieser des Wirbels verstellt werden. Nehmen die einzelnen Korrekturplatten die gewünschte Lage ein, dann wird ein Fixierstab 144 in die einzelnen Aufnahmevorrichtungen 113 der Knochenplatten 101 eingelegt und mit der Fixiereinrichtung 120, die zunächst eingehängt und dann mit der Schraube 127 befestigt wird, fixiert. Nachdem die einzelnen Knochenplatten 101 untereinander fixiert worden sind, werden die Muttern des Korrekturstabes gelöst, der Korrekturstab 145 abgenommen und die einzelnen Korrekturinstrumente 136 abgezogen. Der Korrekturstab 145 und die Korrekturinstrumente 136 stellen also kein Implantat dar, welches im Körper des Patienten verbleibt.

Die erfindungsgemäße Haltevorrichtung weist eine extrem geringe Bauhöhe auf und ist verglichen zu herkömmlichen Haltevorrichtungen leicht und dennoch stabil.

## Patentansprüche

1. Knochenchirurgische Haltevorrichtung für einen Fixierstab, mit einer am Knochen befestigbaren Knochenplatte (1), einer Aufnahmevorrichtung (13) für den Fixierstab und einer den Fixierstab in der Aufnahmevorrichtung (13) haltenden Fixiereinrichtung (20,24,25), dadurch gekennzeichnet, daß die Knochenplatte (1) eine der Knochenoberfläche im wesentlichen angepaßte Anlagefläche (34) aufweist und daß die Aufnahmevorrichtung (13) einstückig mit der Knochenplatte (1) ausgebildet ist, wobei die Aufnahmevorrichtung (13) und die Fixiereinrichtung ein zweigeteiltes Lager bilden, so daß der Fixierstab nach der Befestigung der Knochenplatte (1) am Knochen in die Aufnahmevorrichtung (13) einlegbar und darin fixierbar ist.

2. Knochenchirurgische Haltevorrichtung nach Anspruch 1, dadurch gekennzeichnet, daß an dieser ein zweiter Fixierstab befestigbar ist.

3. Knochenchirurgische Haltevorrichtung nach Anspruch 1 oder 2, dadurch gekennzeichnet, daß die Aufnahmevorrichtung (13) gabelförmig ausgebildet ist und zwei distal abstehende Schenkel (11 und 12) aufweist.

4. Knochenchirurgische Haltevorrichtung nach einem der vorhergehenden Ansprüche, dadurch gekennzeichnet, daß die Aufnahmevorrichtung (13) nach distal offen ist.

5. Knochenchirurgische Haltevorrichtung nach einem der vorhergehenden Ansprüche, dadurch gekennzeichnet, daß sie mit einer Werkzeugangriffseinrichtung (31) versehen ist, an der ein Werkzeug zur Positionierung der Knochenplatte (1) ansetzbar ist.

6. Knochenchirurgische Haltevorrichtung nach Anspruch 5, dadurch gekennzeichnet, daß die Werkzeugangriffseinrichtung (31) von zwei parallelen Flächen (32 und 33) gebildet wird.

7. Knochenchirurgische Haltevorrichtung nach Anspruch 6, dadurch gekennzeichnet, daß die Flächen (32 und 33) an der Aufnahmevorrichtung (13) vorgesehen sind.

8. Knochenchirurgische Haltevorrichtung nach Anspruch 6 oder 7, dadurch gekennzeichnet, daß auf die Flächen (32 und 33) ein gabelförmiges Werkzeug aufschiebbar und nach der Lagekorrektur der Knochenplatte (1) abziehbar ist.

9. Knochenchirurgische Haltevorrichtung nach einem der Ansprüche 3 bis 8, dadurch gekennzeichnet, daß jeder Schenkel (11 bzw. 12) von zwei Fingern (16 und 17 bzw. 18 und 19) gebildet wird, die als Führung für eine Fixierplatte (20) dienen.

10. Knochenchirurgische Haltevorrichtung nach Anspruch 9, dadurch gekennzeichnet, daß zwischen den Fingern (16 bis 19) ein Freiraum besteht, in dem die Fixierplatte (20) in Richtung auf die Knochenplatte (1) beweglich geführt ist.

11. Knochenchirurgische Haltevorrichtung nach Anspruch 9 oder 10, dadurch gekennzeichnet, daß die Finger (16 bis 19) die Fixierplatte (20) in horizontaler Ebene fixieren.

12. Knochenchirurgische Haltevorrichtung nach einem der Ansprüche 9 bis 11, dadurch gekennzeichnet, daß die Fixierplatte (20) über Befestigungseinrichtungen an der Knochenplatte (1) bzw. auf der Knochenplatte (1) befestigbar ist.

13. Knochenchirurgische Haltevorrichtung nach Anspruch 12, dadurch gekennzeichnet, daß die Befestigungseinrichtungen von wenigstens einer, insbesondere von zwei Schrauben (25) gebildet werden.

14. Knochenchirurgische Haltevorrichtung nach Anspruch 13, dadurch gekennzeichnet, daß die Schrauben (25) unverlierbar an der Fixierplatte (20) befestigt sind.

15. Knochenchirurgische Haltevorrichtung nach Anspruch 13 oder 14, dadurch gekennzeichnet, daß die Schrauben (25) in der Längsachse der Fixierplatte (20) angeordnet sind.

16. Knochenchirurgische Haltevorrichtung nach einem der Ansprüche 9 bis 15, dadurch gekennzeichnet, daß die Fixierplatte (20) an ihrer dem Fixierstab zugewandten Seite (30) wenigstens abschnittsweise eine Profilierung aufweist.

17. Knochenchirurgische Haltevorrichtung nach einem der vorhergehenden Ansprüche, dadurch gekennzeichnet, daß die Aufnahmevorrichtung (13) zur Aufnahme eines insbesonderes längsgenuteten Fixierstabes mit einem Durchmesser von 6 mm ausgebildet ist.

18. Knochenchirurgische Haltevorrichtung nach einem der vorhergehenden Ansprüche, dadurch gekennzeichnet, daß die Aufnahmevorrichtung (13) zwischen zwei Befestigungsbohrungen (2 und 3), über die die Knochenplatte (1) am Knochen befestigbar ist, vorgesehen ist.

19. Knochenchirurgische Haltevorrichtung nach Anspruch 18, dadurch gekennzeichnet, daß jede Befestigungsbohrung (2 und 3) einen konischen Sitz (7) für Knochenschrauben (4 und 5) aufweist.

20. Knochenchirurgische Haltevorrichtung nach einem der vorhergehenden Ansprüche, dadurch gekennzeichnet, daß die Knochenplatte (1) spiegelsymmetrisch aufgebaut ist.

21. Knochenchirurgische Haltevorrichtung nach einem der vorhergehenden Ansprüche, dadurch gekennzeichnet, daß die Knochenplatte (101) eine Verbindungseinrichtung für ein zum Beispiel wenigstens einen Korrekturstab (145) enthaltendes Korrekturinstrument (136) aufweist.

22. Knochenchirurgische Haltevorrichtung nach Anspruch 21, dadurch gekennzeichnet, daß das Korrekturinstrument (136) in die Verbindungseinrichtung einschiebbar oder auf diese aufschiebbar ist.

23. Knochenchirurgische Haltevorrichtung nach Anspruch 22, dadurch gekennzeichnet, daß die Einschubrichtung im wesentlichen der Längsrichtung der Knochenplatte (101) entspricht.

24. Knochenchirurgische Haltevorrichtung nach Anspruch 22 oder 23, dadurch gekennzeichnet, daß das Korrekturinstrument (136) einen als Einschubbegrenzer dienenden Anschlag aufweist.

25. Knochenchirurgische Haltevorrichtung nach einem der Ansprüche 21 bis 24, dadurch gekennzeichnet, daß die Verbindungseinrichtung von einem ersten, am Korrekturinstrument (136) vorgesehenen Teil (Vorsprung 138) und einem zweiten, an der Knochenplatte vorgesehenen Teil (Längsnut 137) gebildet wird, wobei die beiden Teile zum Beispiel eine Nut-Feder-Verbindung oder eine Loch-Zapfen-Verbindung bilden.

26. Knochenchirurgische Haltevorrichtung nach einem der Ansprüche 21 bis 25, dadurch gekennzeichnet, daß die Verbindungseinrichtung an der Aufnahmevorrichtung (113) für den Fixierstab (144) vorgesehen ist.

27. Knochenchirurgische Haltevorrichtung nach einem der Ansprüche 21 bis 26, dadurch gekennzeichnet, daß die Verbindungseinrichtung von zwei paralellen Flächen (134, 135) gebildet wird.

28. Knochenchirurgische Haltevorrichtung nach Anspruch 27, dadurch gekennzeichnet, daß wenigstens eine der Fläche mit einer in Einschubrichtung verlaufenden Nut (137) bzw. Vorsprung versehen ist, die bzw. der als Führung für das Korrekturinstrument (136) dient.

29. Knochenchirurgische Haltevorrichtung nach einem der Ansprüche 21 bis 28, dadurch gekennzeichnet, daß die Verbindungseinrichtung eine eindimensionale Relativbewegung zwischen Korrekturinstrument (136) und Knochenplatte (101) erlaubt.

30. Knochenchirurgische Haltevorrichtung nach einem der Ansprüche 21 bis 29, dadurch gekennzeichnet, daß das Korrekturinstrument (136) im wesentlichen gabelförmig ausgebildet ist, wobei bei aufgeschobenem bzw. aufgesetztem Korrekturinstrument (136) die Gabelschenkel (139) die Aufnahmevorrichtung (113) für den Fixierstab (144) umgreifen bzw. flankieren.

31. Knochenchirurgische Haltevorrichtung nach einem der Ansprüche 29 bis 30, dadurch gekennzeichnet, daß in das Korrekturinstrument (136) ein Korrekturstab (145) einlegbar und befestigbar ist.

32. Knochenchirurgische Haltevorrichtung nach einem der vorhergehenden Ansprüche, dadurch gekennzeichnet, daß die Aufnahmevorrichtung (113) einem im wesentlichen rechteckförmigen Grundriß aufweist.

33. Knochenchirurgische Haltevorrichtung nach Anspruch 32, dadurch gekennzeichnet, daß die Längsachse (133) der Aufnahmevorrichtung (113) zur Längsachse (107) der Knochenplatte (101) einen von 0° verschiedenen Winkel aufweist.

34. Knochenchirurgische Haltevorrichtung nach einem der vorhergehenden Ansprüche, dadurch gekennzeichnet, daß die Längsachse (107) der Knochenplatte (101) zur Längsachse des in die Aufnahmevorrichtung (113) einsetzbaren Fixierstabes einen von 90° abweichenden Winkel aufweist.

35. Knochenchirurgische Haltevorrichtung nach einem der vorhergehenden Ansprüche, dadurch gekennzeichnet, daß der Grund (114) der Aufnahmevorrichtung (113) und die zum Knochen distale Oberfläche der Knochenplatte (101) wenigstens abschnittsweise im wesentlichen in der gleichen Ebenen liegen.

36. Knochenchirurgische Haltevorrichtung nach einem der vorhergehenden Ansprüche, dadurch gekennzeichnet, daß die Fixiereinrichtung (120) für den Fixierstab (144) wenigstens ein Lager (Hinterschneidung 122) für wenigstens einen Haken (Vorsprung 121) der Aufnahmevorrichung (113) aufweist und daß die Fixiereinrichtung (120) an der Aufnahmevorrichtung (113) einhängbar ist.

37. Knochenchirurgische Haltevorrichtung nach einem der vorhergehenden Ansprüche, dadurch gekennzeichnet, daß die Fixiereinrichtung (120) für den Fixierstab (144) an der Aufnahmevorrichtung (113) anschraubbar ist.

38. Knochenchirurgische Haltevorrichtung nach einem der vorhergehenden Ansprüche, dadurch gekennzeichnet, daß die Fixiereinrichtung (120) als einhängbarer Klemmdeckel ausgebildet ist.

39. Knochenchirurgische Haltevorrichtung nach einem der vorhergehenden Ansprüche, dadurch gekennzeichnet, daß die Knochenplatte (101) zwei im wesentlichen diagonal angeordnete Befestigungsbohrungen (102 und 103) aufweist.

40. Knochenchirurgische Haltevorrichtung nach einem der vorhergehenden Ansprüche, dadurch gekennzeichnet, daß die Fixiereinrichtung (120) von der Aufnahmevorrichtung (113) bzw. von der Knochenplatte (101) entfernbar ist.

41. Knochenchirurgische Haltevorrichtung nach einem der vorhergehenden Ansprüche, dadurch gekennzeichnet, daß die Fixiereinrichtung (120) über ein Lager mit der Aufnahmevorrichtung (113) bzw. der Knochenplatte (101) verbunden ist.

42. Knochenchirurgische Haltevorrichtung nach Anspruch 41, dadurch gekennzeichnet, daß der zweite Fixierstab an der Knochenplatte (101), an der Aufnahmevorrichtung (113) oder an einer Knochenschraube befestigbar ist.

## Claims

1. A bone surgery holding device for a fixing rod, with a bone plate (1) which can be fastened to the bone, a receiving device (13) for the fixing rod, and a fixing device which secures the fixing rod in the receiving device (13), characterized in that the bone plate (1) has a contact face (34) which is essentially made to fit the surface of the bone and that the receiving device (13) is embodied as being in one piece with the bone plate (1), the receiving device (13) and the fixing device are embodied as a two part bearing resulting in that after the fastening of the bone plate (1) to the bone, the fixing rod can be inserted and fixed in the receiving device (13).

2. The bone surgery holding device according to claim 1, characterized in that a second fixing rod can be fastened onto it.

3. The bone surgery holding device according to claim 1 or 2, characterized in that the receiving device (13) is embodied as fork-shaped and has two distally protruding legs (11 and 12).

4. The bone surgery holding device according to one of the preceding claims, characterized in that the receiving device (13) is open distally.

5. The bone surgery holding device according to one of the preceding claims, characterized in that it is provided with a tool engaging device (31) on which a tool can be placed to position the bone plate (1).

6. The bone surgery holding device according to claim 5, characterized in that the tool engaging device (31) is embodied by two parallel faces (32 and 33).

7. The bone surgery holding device according to claim 6, characterized in that the faces (32 and 33) are provided on the receiving device (13).

8. The bone surgery holding device according to claim 6 or 7, characterized in that a fork-shaped tool can be slipped onto the faces (32 and 33) and removed after the position correction of the bone plate (1).

9. The bone surgery holding device according to one of claims 3-8, characterized in that each leg (11 or 12) is constituted of two prongs (16 and 17 or 18 and 19), which serve as a guide for a fixing plate (20).

10. The bone surgery holding device according to claim 9, characterized in that an empty space exists between the prongs (16-19), in which space the fixing plate (20) is guided so that it can move in the direction of the bone plate (1).

11. The bone surgery holding device according to claim 9 or 10, characterized in that the prongs (16-19) fix the fixing plate (20) in the horizontal plane.

12. The bone surgery holding device according to one of claims 9-11, characterized in that the fixing plate (20) can be fastened to the bone plate (1) or on the bone plate (1) via fastening devices.

13. The bone surgery holding device according to claim 12, characterized in that the fastening devices are constituted of two screws (25).

14. The bone surgery holding device according to claim 13, characterized in that the screws (25) are fastened to the fixing plate (20) in captive fashion.

15. The bone surgery holding device according to claim 13 or 14, characterized in that the screws (25) are disposed in the longitudinal axis of the fixing plate (20).

16. The bone surgery holding device according to one of claims 9-15, characterized in that the fixing plate (20), at least in part, has a profiling on its side (30) oriented toward the fixing rod.

17. The bone surgery holding device according to one of the preceding claims, characterized in that the receiving device (13) is embodied to receive an especially longitudinally grooved fixing rod with a diameter of 6 mm.

18. The bone surgery holding device according to one of the preceding claims, characterized in that the receiving device (13) is provided between two fastening bores (2 and 3), via which the bone plate (1) can be fastened on the bone.

19. The bone surgery holding device according to claim 18, characterized in that each fastening bore (2 and 3) has a conical seat (7) for bone screws (4 and 5).

20. The bone surgery holding device according to one of the preceding claims, characterized in that the bone plate (1) is constructed mirror symmetrically.

21. The bone surgery holding device according to one of the preceding claims, characterized in that the bone plate (101) has a connecting device for a correction instrument (136), which includes at least one correction rod (145).

22. The bone surgery holding device according to claim 21, characterized in that the correction instrument (136) can be slipped into or onto the connecting device.

23. The bone surgery holding device according to claim 22, characterized in that the insertion direction essentially corresponds to the longitudinal direction of the bone plate (101).

24. The bone surgery holding device according to claim 22 or 23, characterized in that the correction instrument (136) has a stop that serves as an insertion limiter.

25. The bone surgery holding device according to one of claims 21-24, characterized in that the connecting device is constituted by a first part (projection 138) provided on the correction instrument (136) and a second part (longitudinal groove 137) provided on the bone plate, wherein the two parts constitute for example a tongue-and-groove connection or a hole-and-peg connection.

26. The bone surgery holding device according to one of claims 21-25, characterized in that the connecting device is provided on the receiving device (113) for the fixing rod (144).

27. The bone surgery holding device according to one of claims 21-26, characterized in that the connecting device is constituted of two parallel faces (134, 135).

28. The bone surgery holding device according to claim 27, characterized in that at least one of the faces is provided with a groove (137) or projection extending in the insertion direction, which serves as a guide for the correction instrument (136).

29. The bone surgery holding device according to one of claims 21-28, characterized in that the connecting device allows a one-dimensional relative movement between the correction instrument (136) and the bone plate (101).

30. The bone surgery holding device according to one of claims 21-29, characterized in that the correction instrument (136) is embodied as essentially fork-shaped, wherein when the correction instrument (136) is slipped on or placed, the fork legs (139) encompass or flank the receiving device (113) for the fixing rod (144).

31. The bone surgery holding device according to one of claims 21-30, characterized in that a correction rod (145) can be inserted into and fastened in the correction instrument (136).

32. The bone surgery holding device according to one of the preceding claims, characterized in that the receiving device (113) has an essentially rectangle-shaped outline.

33. The bone surgery holding device according to claim 32, characterized in that the longitudinal axis (133) of the receiving device (113) differs from the longitudinal axis (107) of the bone plate (101) by an angle other than 0°.

34. The bone surgery holding device according to one of the preceding claims, characterized in that the longitudinal axis (107) of the bone plate (101) diverges by an angle other than 90° from the longitudinal axis of the fixing rod, which can be inserted into the receiving device (113).

35. The bone surgery holding device according to one of the preceding claims, characterized in that the bottom (114) of the receiving device (113) and the surface of the bone plate (101) distal to the bone, at least in part, lie essentially in the same plane.

36. The bone surgery holding device according to one of the preceding claims, characterized in that the fixing device (120) for the fixing rod (144) has at least one support (undercut 122) for at least one hook (projection 121) of the receiving device (113) and that the fixing device (120) can be suspended from the receiving device (113).

37. The bone surgery holding device according to one of the preceding claims, characterized in that the fixing device (120) for the fixing rod (144) can be screwed onto the receiving device (113).

38. The bone surgery holding device according to one of the preceding claims, characterized in that the fixing device (120) is embodied as a suspendible clamping cover.

39. The bone surgery holding device according to one of the preceding claims, characterized in that the bone plate (101) has two diagonally disposed fastening bores (102 and 103).

40. The bone surgery holding device according to one of the preceding claims, characterized in that the fixing device (120) can be removed from the receiving device (113) or the bone plate (101).

41. The bone surgery holding device according to one of the preceding claims, characterized in that the fixing device (120) is connected via a support to the receiving device (113) or the bone plate (101).

42. The bone surgery holding device according to one of the preceding claims, characterized in that the second fixing rod can be fastened on the bone plate (101), the receiving device (113), or a bone screw.

## Revendications

1. Fixateur pour chirurgie osseuse d'une tige de fixation, pourvu d'une plaque d'ostéosynthèse (1) apte à être fixée sur l'os, d'un élément récepteur (13) d'une tige de fixation, et d'un dispositif de fixation (20, 24, 25) maintenant la tige de fixation dans l'élément récepteur (13), caractérisé en ce que la plaque d'ostéosynthèse (1) présente une face d'appui (34) essentiellement adaptée à la surface de l'os, et en ce que l'élément récepteur (13) est réalisé d'un seul tenant avec la plaque d'ostéosynthèse (1), l'élément récepteur (13) et le dispositif de fixation formant un logement bipartite, de telle sorte que la tige de fixation peut être insérée dans l'élément récepteur (13) et y être fixée, après fixation de la plaque d'ostéosynthèse (1) sur l'os.

2. Fixateur pour chirurgie osseuse selon la revendication 1, caractérisé en ce qu'une deuxième tige de fixation peut lui être fixée.

3. Fixateur pour chirurgie osseuse selon la revendication 1 ou 2, caractérisé en ce que l'élément récepteur (13) est conçu en forme de fourche et présente deux branches distales (11 et 12) en saillie.

4. Fixateur pour chirurgie osseuse selon l'une des revendications précédentes, caractérisé en ce que l'élément récepteur (13) est ouvert à son extrémité distale.

5. Fixateur pour chirurgie osseuse selon l'une des revendications précédentes, caractérisé en ce qu'il est doté d'un agencement (31) formant empreinte pour un outil et susceptible de recevoir un outil en vue du positionnement de la plaque d'ostéosynthèse (1).

6. Fixateur pour chirurgie osseuse selon la revendication 5, caractérisé en ce que l'agencement (31) formant empreinte pour l'outil est formé par deux faces (32 et 33) parallèles.

7. Fixateur pour chirurgie osseuse selon la revendication 6, caractérisé en ce que les faces (32 et 33) sont prévues sur l'élément récepteur (13).

8. Fixateur pour chirurgie osseuse selon l'une des revendications 6 ou 7, caractérisé en ce qu'un outil fourchu peut être glissé sur les faces (32 et 33) et retiré après correction de la position de la plaque d'ostéosynthèse (1).

9. Fixateur pour chirurgie osseuse selon l'une des revendications 3 à 8, caractérisé en ce que chaque branche (11, 12) est formée de deux doigts (16 et 17, 18 et 19) assurant le guidage d'une plaque de fixation (20).

10. Fixateur pour chirurgie osseuse selon la revendication 9, caractérisé en ce qu'entre les doigts (16 à 19) est défini un espace vide dans lequel la plaque de fixation (20) est guidée mobile en direction de la plaque d'ostéosynthèse (1).

11. Fixateur pour chirurgie osseuse selon la revendication 9 ou 10, caractérisé en ce que les doigts (16 à 19) maintiennent la plaque de fixation (20) dans un plan horizontal.

12. Fixateur pour chirurgie osseuse selon l'une des revendications 9 à 11, caractérisé en ce que la plaque de fixation (20) est apte à être fixée à la plaque d'ostéosynthèse (1) ou sur la plaque d'ostéosynthèse (1) au moyen de dispositifs de fixation.

13. Fixateur pour chirurgie osseuse selon la revendication 12, caractérisé en ce que les dispositifs de fixation sont constitués d'au moins une, et plus particulièrement de deux vis (25).

14. Fixateur pour chirurgie osseuse selon la revendication 13, caractérisé en ce que les vis (25) sont fixées imperdables à la plaque de fixation (20).

15. Fixateur pour chirurgie osseuse selon la revendication 13 ou 14, caractérisé en ce que les vis (25) sont agencées sur l'axe longitudinal de la plaque de fixation (20).

16. Fixateur pour chirurgie osseuse selon l'une des revendications 9 à 15, caractérisé en ce que la plaque de fixation (20) est au moins partiellement profilée sur son côté (30) en regard de la tige de fixation.

17. Fixateur pour chirurgie osseuse selon l'une des revendications précédentes, caractérisé en ce que l'élément récepteur (13) est conçu pour recevoir une tige de fixation, notamment une tige de fixation rainurée longitudinalement, d'un diamètre de 6 mm.

18. Fixateur pour chirurgie osseuse selon l'une des revendications précédentes, caractérisé en ce que l'élément récepteur (13) est prévu entre deux alésages de fixation (2,3), permettant de fixer la plaque d'ostéosynthèse (1) sur l'os.

19. Fixateur pour chirurgie osseuse selon la revendication 18, caractérisé en ce que chaque alésage de fixation (2,3) présente un siège (7) conique apte à recevoir une vis pour ostéosynthèse (4,5).

20. Fixateur pour chirurgie osseuse selon l'une des revendications précédentes, caractérisé en ce que la plaque d'ostéosynthèse (1) est de conception à symétrie spéculaire.

21. Fixateur pour chirurgie osseuse selon l'une des revendications précédentes, caractérisé en ce que la plaque d'ostéosynthèse (101) présente un dispositif de raccordement pour un instrument de correction (136) contenant par exemple au moins une tige de correction (145).

22. Fixateur pour chirurgie osseuse selon la revendication 21, caractérisé en ce que l'instrument de correction (136) peut être inséré dans le dispositif de raccordement ou engagé sur ledit dispositif.

23. Fixateur pour chirurgie osseuse selon la revendication 22, caractérisé en ce que la direction d'insertion coïncide sensiblement avec la direction longitudinale de la plaque d'ostéosynthèse (101).

24. Fixateur pour chirurgie osseuse selon la revendication 22 ou 23, caractérisé en ce que l'instrument de correction (136) présente une butée servant de limite d'insertion.

25. Fixateur pour chirurgie osseuse selon l'une des revendications 21 à 24, caractérisé en ce que le dispositif de raccordement est constitué d'une première partie prévue sur l'instrument de correction (136) (saillie 138) et d'une deuxième partie prévue sur la plaque d'ostéosynthèse (rainure longitudinale 137), les deux parties formant par exemple un assemblage à rainure-languette ou un assemblage à tourillons.

26. Fixateur pour chirurgie osseuse selon l'une des revendications 21 à 25, caractérisé en ce que le dispositif de raccordement est prévu sur l'élément récepteur (113) de la tige de fixation (144).

27. Fixateur pour chirurgie osseuse selon l'une des revendications 21 à 26, caractérisé en ce que le dispositif de raccordement est constitué de deux faces (134, 135) parallèles.

28. Fixateur pour chirurgie osseuse selon la revendication 27, caractérisé en ce qu'au moins une des faces est pourvue d'une rainure (137) ou d'une saillie ménagée dans la direction d'insertion, ladite rainure ou ladite saillie assurant le guidage de l'instrument de correction (136).

29. Fixateur pour chirurgie osseuse selon l'une des revendications 21 à 28, caractérisé en ce que le dispositif de raccordement permet un mouvement relatif unidimensionnel de l'instrument de correction (136) par rapport à la plaque d'ostéosynthèse (101).

30. Fixateur pour chirurgie osseuse selon l'une des revendications 21 à 29, caractérisé en ce que l'instrument de correction (136) est essentiellement conçu en forme de fourche, les branches de la fourche (139) enserrant ou flanquant l'élément récepteur (113) de la tige de fixation (144) lorsque l'instrument de correction (136) est engagé ou encastré.

31. Fixateur pour chirurgie osseuse selon l'une des revendications 29 à 30, caractérisé en ce qu'une tige de correction (145) peut être introduite et fixée dans l'instrument de correction (136).

32. Fixateur pour chirurgie osseuse selon l'une des revendications précédentes, caractérisé en ce que l'élément récepteur (113) présente un contour sensiblement rectangulaire.

33. Fixateur pour chirurgie osseuse selon la revendication 32, caractérisé en ce que l'axe longitudinal (133) de l'élément récepteur (113) et l'axe longitudinal (107) de la plaque d'ostéosynthèse (101) forment un angle différent de 0°.

34. Fixateur pour chirurgie osseuse selon l'une des revendications précédentes, caractérisé en ce que l'axe longitudinal (107) de la plaque d'ostéosynthèse (101) et l'axe longitudinal de la tige de fixation pouvant être insérée dans l'élément récepteur (113) forment un angle différent de 90°.

35. Fixateur pour chirurgie osseuse selon l'une des revendications précédentes, caractérisé en ce que le fond (114) de l'élément récepteur (113) et la face opposée à l'os de la plaque d'ostéosynthèse (101) sont, au moins partiellement, sensiblement coplanaires.

36. Fixateur pour chirurgie osseuse selon l'une des revendications précédentes, caractérisé en ce que le dispositif de fixation (120) de la tige de fixation (144) présente au moins un appui (contre-dépouille 122) apte à recevoir au moins un crochet (saillie 121) que présente l'élément récepteur (113), et en ce que le dispositif de fixation (120) est apte à être enclipsé sur l'élément récepteur (113).

37. Fixateur pour chirurgie osseuse selon l'une des revendications précédentes, caractérisé en ce que le dispositif de fixation (120) de la tige de fixation (144) est apte à être vissé à l'élément récepteur (113).

38. Fixateur pour chirurgie osseuse selon l'une des revendications précédentes, caractérisé en ce que le dispositif de fixation (120) est conçu sous forme d'un couvercle de serrage enclipsable.

39. Fixateur pour chirurgie osseuse selon l'une des revendications précédentes, caractérisé en ce que la plaque d'ostéosynthèse (101) présente deux alésages de fixation (102 et 103) agencés sensiblement sur sa diagonale.

40. Fixateur pour chirurgie osseuse selon l'une des revendications précédentes, caractérisé en ce que le dispositif de fixation (120) peut être retiré de l'élément récepteur (113) ou de la plaque d'ostéosynthèse (101).

41. Fixateur pour chirurgie osseuse selon l'une des revendications précédentes, caractérisé en ce que le dispositif de fixation (120) est relié à l'élément récepteur (113) ou à la plaque d'ostéosynthèse (101) par l'intermédiaire d'un appui.

42. Fixateur pour chirurgie osseuse selon la revendication 41, caractérisé en ce que la deuxième tige de fixation est apte à être fixée à la plaque d'ostéosynthèse (101), à l'élément récepteur (113) ou à une vis pour ostéosynthèse.
